(19) Europäisches Patentamt / European Patent Office / Office européen des brevets

(11) **EP 3 354 196 A1**

(12) **EUROPEAN PATENT APPLICATION**
published in accordance with Art. 153(4) EPC

(43) Date of publication:
**01.08.2018 Bulletin 2018/31**

(51) Int Cl.:
**A61B 5/026** (2006.01)

(21) Application number: **16848673.6**

(22) Date of filing: **23.09.2016**

(86) International application number:
**PCT/JP2016/078050**

(87) International publication number:
**WO 2017/051885 (30.03.2017 Gazette 2017/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **25.09.2015 JP 2015188714**

(71) Applicant: **NeU Corporation
Tokyo 101-0048 (JP)**

(72) Inventors:
• **HASEGAWA, Kiyoshi
Tokyo 105-8717 (JP)**
• **KATURA, Takusige
Tokyo 105-8717 (JP)**

(74) Representative: **Strehl Schübel-Hopf & Partner
Maximilianstrasse 54
80538 München (DE)**

(54) **INFORMATION PROCESSING DEVICE, INFORMATION PROCESSING METHOD AND INFORMATION PROCESSING PROGRAM**

(57) This information processing device is provided with: a receiving unit which receives a detected value detected by means of a head-mounted device which is worn on the head of a user and detects the blood flow rate in the head; a calculating unit which extracts a first signal by passing the detected value through a first filter which extracts a signal in a first frequency band, extracts a second signal by passing the detected value through a second filter which extracts a signal in a second frequency band, which is a frequency band that is higher than the first frequency band, and extracts a third signal, which is the envelope of the second signal, from the second signal; and an output unit which outputs the first signal and the third signal in association with one another.

FIG. 1

## Description

TECHNICAL FIELD

The present invention pertains to an information processing apparatus, an information processing method, and an information processing program.

BACKGROUND ART

Generally, a good circulation of blood of a human brain (an increase in bloodflow rate) is deemed important for health. The bloodflow rate increases as a cardiac rate rises. However, exercises and other equivalent movements cause the rise in cardiac rate, while blood vessels get contracted due to enhancement of activities of sympathetic nerves. The contraction of the blood vessel does not lead to the increase in bloodflow rate. It is therefore desirable to increase the bloodflow rate, while restraining the activities of the sympathetic nerves.

[Documents of Prior Arts]

[Patent Documents]

[Patent Document 1] Japanese Patent Application Laid-Open Publication No. 2000-37558.

SUMMARY OF THE INVENTION

[Problems to be solved by the Invention]

The activities of the sympathetic nerves depend on variations of pulse wave. It is considered that an amplitude of the pulse wave decreases in a state where the activities of the sympathetic nerves become active but increase in a state where the activities of the sympathetic nerves become inactive (relaxed).

It is requested to perform training for increasing the bloodflow rate in a relaxed state by observing the variations of the bloodflow rate and the variations of the amplitude of the pulse wave. In other words, it is requested to make a phase of the variation of the bloodflow rate coincident with a phase of the variation of the amplitude of the pulse wave. It is, however, difficult to simply observe the variation of the bloodflow rate and the variation of the amplitude of the pulse wave together with a phase difference between both of the variations, and it is therefore difficult to measure an effect even by performing the training.

The present invention aims at providing an apparatus configured to output in a way that makes recognizable a phase difference between a variation of bloodflow rate and a variation of amplitude of a pulse wave.

[Means for solving the Problems]

Means given below are adopted for solving the problems described above.

To be specific, a first aspect is an information processing device including: a receiving unit to receive a detection value detected by a head region attaching device, attached to a head region of a user, to detect a bloodflow rate of the head region; a calculation unit to extract a first signal by filtering the detection value with a first filter to extract a signal of a first frequency band, to extract a second signal by filtering the detection value with a second filter to extract a signal of a second frequent band higher than the first frequency band, and to extract a third signal as an envelope of the second signal from the second signal; and an output unit to output the first signal and the third signal by being associated with each other.

An aspect of the disclosure may be actualized such that an information processing apparatus runs a program. In other words, a configuration of the disclosure may be specified as a program run by an information processing apparatus that causes the respective means to execute processes in the aspect described above, or as a non-transitory computer readable recording medium on which this program is recorded. The configuration of the disclosure may also be specified as a method carried out by the information processing apparatus that causes the respective means to execute the processes. The configuration of the disclosure may further be specified as a system including the information processing apparatus that causes the respective means to execute the processes.

Steps describing the program contain, as a matter of course, the processes to be executed in time-series along a written sequence, and also processes that are not necessarily executed in time-series but executed in parallel or individually. Part of the steps describing the program may also be omitted.

[Effect of the Invention]

According to the present invention, it is possible to provide an apparatus configured to make recognizable the phase difference between the variation of the bloodflow rate and the variation of the amplitude of the pulse wave.

BRIEF DESCRIPTION OF THE DRAWINGS

FIG. 1 is a diagram illustrating a configuration concerning information processing of a measurement system according to one embodiment of the present invention.
FIG. 2 is a diagram illustrating an example of a configuration of a head region attaching device.
FIG. 3 is a diagram illustrating an example of a configuration of a user terminal.
FIG. 4 is a diagram illustrating an example of a configuration of a server.

FIG. 5 is a flowchart illustrating an example of an operation flow of the measurement system according to the embodiment.

FIG. 6 is a graphic chart depicting examples of a waveform of brain activity, a signal of a variation component of a bloodflow rate, and a signal of a component of a pulse wave.

FIG. 7 is a graphic chart depicting examples of a signal of a component of the pulse wave, and a signal of a variation component of an amplitude of the pulse wave.

FIG. 8 is a graphic chart depicting examples of a signal of a variation component of the bloodflow rate and a signal of a variation component of the amplitude of the pulse wave, which are outputted to an output unit of a user terminal.

DESCRIPTION OF THE EMBODIMENTS

An embodiment will hereinafter be described with reference to the drawings. A configuration of the embodiment is an exemplification, and a configuration of the invention is not limited to a specific configuration of the embodiment. When carrying out the invention, specific configurations corresponding to embodiments may also be properly adopted.

There is a measurement system, in which a head region attaching device called a headset is provided with a near infrared ray irradiation unit and a near infrared ray detection unit to thereby detect variations in bloodflow rate on a surface of a brain, and a data processing apparatus (user terminal) processes detected data. This measurement system is capable of measuring states of how the bloodflow rate increases and decreases. Measurement signals of this measurement system are overlapped with pulse wave components. Amplitude of the pulse wave components are considered to decrease because of blood vessels getting contracted in an activating state of sympathetic nerves, but considered to increase because of the blood vessels getting flaccid in a relaxing state thereof.

[Embodiment]

(Example of Configuration)

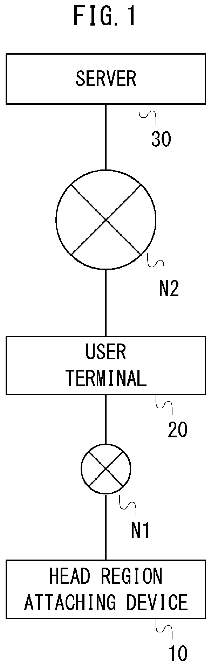

FIG. 1 is a diagram illustrating a configuration concerning information processing of the measurement system according to one embodiment of the present invention. The measurement system detects measurement data (also termed detection values) representing variations in bloodflow rate from a head region of a user, and thus acquires brain activity information (brain activity waveforms) representing activity states of a user's brain.

As in FIG. 1, the measurement system includes a head region attaching device 10, a user terminal 20 and a server 30. The head region attaching device 10 and the user terminal 20 are interconnected via a network N1. The user terminal 20 and the server 30 are interconnected via a network N2.

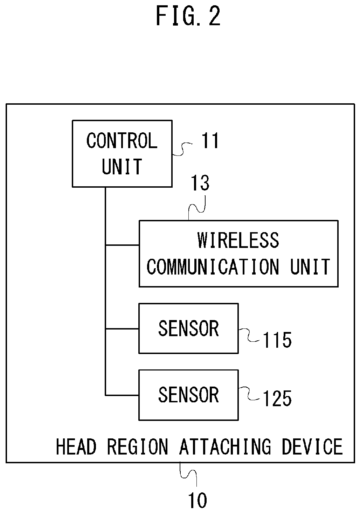

FIG. 2 is a diagram illustrating an example of a configuration of the head region attaching device. The head region attaching device 10 includes, as an aspect of information processing, a control unit 11, a wireless communication unit 13, and a pair of sensors 115, 125. The control unit 11 controls measurements made by and communications with the head region attaching device 10. The control unit 11, which includes a processor instanced by a Central Processing unit (CPU) or a Data Signal Processor (DSP), and a memory, executes processing based on a computer program, firmware and other equivalent software deployed in an executable manner on the memory. However, the control unit 11 may also be a dedicated hardware circuit, a Field Programmable Gate Array (FPGA) and other equivalent circuits configured to execute cooperative processes with respective components by starting up the wireless communication unit 13 and the sensors 115, 125. The control unit 11 may further be a mixed entity of the CPU, the DSP, the dedicated hardware circuit and other equivalent circuits.

The head region attaching device 10 has a structure of being attached to the head region of the user by being wound round the head region in a headband shape, and thus being fixed to the head region of the user by fastening a member for fixation.

The wireless communication unit 13 is connected via a predetermined interface to the control unit 11 and the sensors 115, 125. However, the wireless communication unit 13 may also be configured to acquire the data from the sensors 115, 125 through the control unit 11. The wireless communication unit 13 performs communications with the user terminal 20 via the network N1. The network N1 is a network pursuant to Standards instanced by Bluetooth (registered trademark), wireless Local Area Network (LAN) and ZigBee. The wireless communication unit 13 is one example of transfer means. The measurement system is not, however, limited to the Standards of the wireless interface for the wireless communication unit 13.

When performing the communications via the network N1, an identifier for identifying the head region attaching device 10 is embedded in a header field of a communication header, or in a user's data field (payload field) in communication data, thereby enabling the user terminal 20 to identify the user (measurement examinee).

The measurement system may further be provided with a communication unit that performs wired communications in place of the wireless communication unit 13 or together with the wireless communication unit 13. In other words, the head region attaching device 10 and the user terminal 20 may also be connected together via an interface for the wired communications. In this case, it does not mean that there are limitations to the interface for the wired communications, but a variety of interfaces instanced by Universal Serial Bus (USB) and Peripheral Component Interconnect Express (PCI Express) are usable corresponding to applications of the measurement system.

Each of the sensors 115, 125 irradiates the head region with near infrared rays, then receives the near infrared rays partly absorbed and scattered in the vicinity of a brain cortex of the brain, and converts the received rays into electric signals. The brain cortex of the brain has a bloodflow rate that differs corresponding to, e.g., the activity states of the brain. As a result, in respective parts of the brain cortex of the brain, there occur variations in quantity of haemoglobin bound to oxygen in blood and a quantity of haemoglobin not bounded to the oxygen. An absorption characteristic or a scattering characteristic of the near infrared rays varies in the vicinity of the brain cortex of the brain due to the variations in haemoglobin quantity, variations in oxygen quantity and other equivalent variations. The sensors 115, 125 convert the near infrared rays, into the electric signals, of which a light quantity varies based on variations in absorption rate or variations in transmittance of the near infrared rays, corresponding to such a state of the bloodflow in the vicinity of the brain cortex of the brain, and output the thus-converted electric signals. The sensors 115, 125 are one example of detection means.

Each of the sensors 115, 125 includes, e.g., a near infrared ray light source to irradiate the near infrared rays, and a light receiving unit to receive the near infrared rays. The near infrared ray light source is exemplified by a Light Emitting Diode (LED) and an infrared ray lamp. The light receiving unit includes: a photoelectric element instanced by a photo diode and a photo transistor; an amplifier; and an Analog-to-Digital (AD) converter. Note that the near infrared ray light source and the light receiving unit may not be paired when provided. For example, a plurality of light receiving units may be provided for one near infrared ray light source.

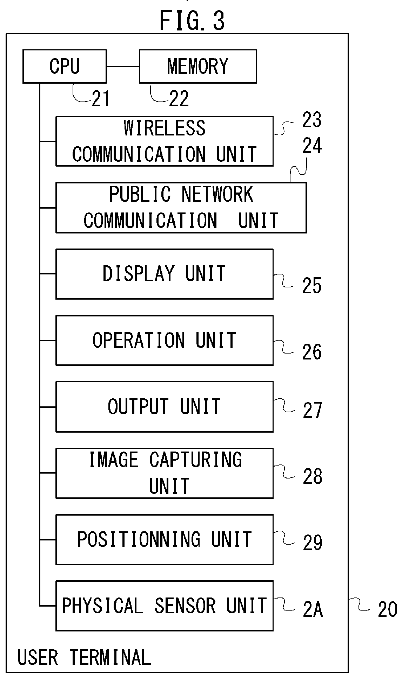

FIG. 3 is a diagram illustrating an example of a configuration of the user terminal. The user terminal 20 acquires, from the head region attaching device 10, variation data of the absorption rate or the transmittance of the near infrared rays in the vicinity of the brain cortex of the user's brain, and provides services including a variety of information processes related to the activity states of the user's brain. The user terminal 20 is one example of an information processing apparatus (computer) . The user terminal 20 may be attained by using a dedicated or general-purpose computer instanced by a Personal Computer (PC), a smartphone, a mobile phone, a tablet terminal, a car navigation system and a Personal Digital Assistant (PDA), or by using electronic equipment mounted with the computer. The user terminal 20 may be installed at, e.g., a fitness club, a cram school and other equivalent places.

The user terminal 20 includes a CPU 21, a memory 22, a wireless communication unit 23, a public network communication unit 24, a display unit 25, an operation unit 26, an output unit 27, an image capturing unit 28, a positioning unit 29, and a physical sensor unit 2A. The CPU 21 executes processing as the user terminal 20, based on a computer program deployed in an executable manner on the memory 22. The processing as the user terminal 20 is, e.g., a service encompassing a variety of information processes related to the activity states of the user's brain. The CPU 21 running such a computer program is one example of calculating means.

The memory 22 stores the computer program run by the CPU 21, or data processed by the CPU 21. The memory 22 may include a volatile memory and a nonvolatile memory.

The wireless communication unit 23 is the same as the wireless communication unit 13 of the head region attaching device 10. The wireless communication unit 23 is one example of receiving means. The user terminal 20 may also include a communication unit to perform the wired communications in place of the wireless communication unit 23 or together with the wireless communication unit 23.

The public network communication unit 24 performs communications via a network N2 with a server, e.g., a server 30 and other equivalent devices on the network N2. The network N2 is a public network, and is exemplified by a mobile phone network. When the network N2 is the mobile phone network, the public network communication unit 24 establishes a connection to the network N2 via a base station of the mobile phone network. However, the network N2 may also be a network including: an access network to a communication apparatus of an Internet provider; and the Internet. The access network to the communication apparatus of the Internet provider is exemplified by an optical network provided by a common carrier, and Asymmetric Digital Subscriber Line (ADSL). The network N2 is one example of a public wireless network. The public network communication unit 24 is one example of public wireless communication means. It does not, however, mean that the network N2 is limited to the public network in the measurement system; and the network N2 may also be an in-house network instanced by a Local Area Network (LAN), a private line of a business enterprise, an entrepreneur, a city hall, a school, a research institution and other equivalent organizations, and a wide area network instanced by a Virtual Private Network (VPN). The business enterprise, the entrepreneur, the cityhall, the school, the research institution and other equivalent organizations will hereinafter be simply referred to as the enterprise and other equivalent organizations .

The display unit 25, which is instanced by a liquid crystal display and an Electro-Luminescence (EL) panel, displays information outputted from the CPU 21. The operation unit 26, which is instanced by a push button and a touch panel, accepts user's operation. The output unit 27 is, e.g., a vibrator to output the vibrations, a loudspeaker to output sounds or voices, and other equivalent devices. The image capturing unit 28 is, e.g., a camera including a solid-state image capturing element. The solid-state image capturing element may involve making use of a Charge-Coupled Device (CCD) image sensor, a Complementary Metal Oxide Semiconductor (CMOS) image sensor, and other equivalent image sensors.

The positioning unit 29, which is instanced by a Global Positioning System (GPS) receiver, receives radio waves from a GPS satellite, thereby calculating a present position (latitude, longitude, and other equivalent geographical coordinates), time and other equivalent data. It does not, however, mean that the positioning unit 29 is limited to a configuration including the GPS receiver. For example, when the public network communication unit 24 is applied to the mobile phone network, the positioning unit 29 may execute positioning based on a distance from the mobile phone base station.

The physical sensor unit 2A is, e.g., an acceleration sensor or an angular acceleration sensor, and other equivalent sensors. The physical sensor unit 2A may, however, be a temperature sensor, a humidity sensor, a barometric pressure sensor or a hydraulic pressure sensor.

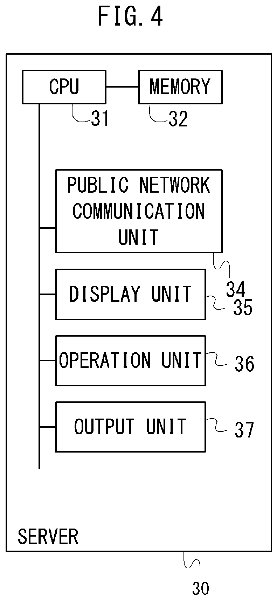

FIG. 4 is a diagram illustrating an example of a configuration of the server. The server 30 is connected to the network N2. The server 30 is a general type of information processing apparatus. The information processing apparatus includes: a CPU to executing arithmetic and control operations; a memory and a storage unit that store data and other equivalent information used for the arithmetic and other equivalent operations; an input unit to accept an input of information given from a user and other equivalent persons; an output unit to output information by images, sounds/voices and other equivalent elements; and a communication unit and other equivalent units to transmitting and receiving the information to and from other apparatuses. The server 30 may be attained by employing a dedicated or a general-purpose computer as instanced by the PC and a workstation (WS), or by employing the electronic equipment mounted with the computer.

The server 30 includes a CPU 31, a memory 32, a public network communication unit 34, a display unit 35, an operation unit 36, and an output unit 37. The CPU 31 executes processing as the server 30, based on a computer program deployed in the executable manner on the memory 32. The processing as the server 30 is, e.g., a service encompassing a variety of information processes related to the activity states of the brain. The CPU 31 running such a computer program is one example of calculating means.

The memory 32 stores the computer program run by the CPU 31, or data processed by the CPU 31. The memory 32 may include a volatile memory and a nonvolatile memory. An individual information table T20 containing individual information of the user is stored in the memory 32.

The public network communication unit 34 performs communications via the network N2 with an equipment, e.g., the user terminal 20 on the network N2. The network N2 is a public network, and is exemplified by a mobile phone network. The public network communication unit 34 is one example of public wireless communication means.

The display unit 35, which is instanced by the liquid crystal display and the Electro-Luminescence (EL) panel, displays information outputted from the CPU 31. The operation unit 36, which is instanced by the push button and the touch panel, accepts the user's operation. The output unit 37 is, e.g., the vibrator to output the vibrations, the loudspeaker to output the sounds or the voices, and other equivalent devices .

<Operational Example>

An operational example of the measurement system according to the embodiment will be described.

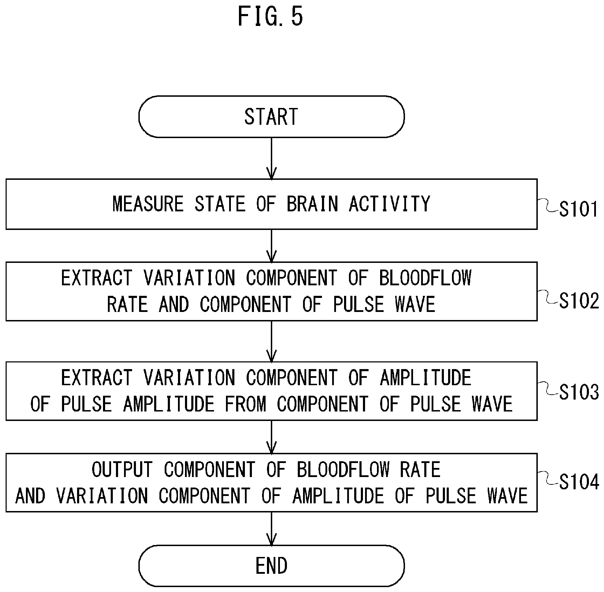

FIG. 5 is a flowchart illustrating an example of an operation flow of the measurement system according to the embodiment. Each head region attaching device 10 of the measurement system according to the embodiment is attached to the head region of the user (measurement examinee), and is in a status enabled to measure the activity states of the brain (bloodflow rates of the brain). Each head region attaching device 10 is connected to the user terminal 20. It is assumed that calibration and other equivalent operations of the head region attaching device 10 are already done. The user terminal 20 is connected to the server 30.
In S101, the user terminal 20 instructs the head region attaching device 10 to measure the activity states of the brain of the user wearing the head region attaching device 10. The activity states of the brain are measured at a predetermined sampling frequency. Each head region attaching device 10 measures the activity states of the brain of the user by the sensors 115, 125, and transmits detection values representing the activity states of the brain to the user terminal 20 via the wireless communication unit 13. The user terminal 20, upon receiving the detection values (waveforms of the brain activities) representing the activity states of the brain from the head region attaching device 10 via the wireless communication unit 23, stores the detection values in a storage means instanced by the memory 22. Herein, the detection value may be a measured value itself, and may also be information processed to facilitate the transmission of the measured value to the user terminal 20, or may also be information into which the values measured for a fixed period are aggregated. It is sufficient that the detection value is a value based on the value obtained when the head region attaching device 10 measures a variation in bloodflow of the head region. A unique identifier is allocated beforehand to each individual user. The detection values representing the activity states of the brain are stored as time-series data together with the identifier of the user.
The user terminal 20 may allow, when measuring the activity states of the brain, the user wearing the head region attaching device 10 to conduct predetermined behaviors. The predetermined behaviors are, e.g., Zen meditation, cogitation, yoga, a sleep, watching a video, playing a game, musical appreciation, an exercise, eating and drinking, a test, and playing an application. The predetermined behaviors may also be specified behaviors in specified applications. These applications include, e.g., a brain training application. For example, the user terminal 20, when made to watch the video, causes the display unit 25 to display a video, and causes the output unit 27 to output sounds/voices accompanied with moving images, thus making the user watch the video. The user terminal 20, when made to play the game, causes the display unit 25 and the output unit 27 to output images and sounds/voices for the game, and causes the user to manipulate the game by use of the operation unit 26, thus making the user play the game. The user terminal 20 outputs signs of starting and finishing a predetermined motion as the predetermined behavior through the display unit 25 and the output unit 27 by the images and the sounds/voices. The user terminal 20 may accept inputs of operations for the signs of starting and finishing the predetermined motion as the predetermined behavior through the input unit 26.

The user terminal 20 transmit, to the server 30, the detection values (the waveforms of the brain activities) representing the activity states of the brain to be stored in the storage means together with the identifier of the user and time information via the public network communication unit 24. The server 30 receives, from each user terminal 20, the detection values representing the activity states of the brain, an identifier of the predetermined behavior, the identifier of the user and the time information via the public network communication unit 34. The server 30 stores the received waveforms of the brain activities by being associated with the identifier (ID) of the user and the time information in the memory 32. The waveforms of the brain activities of a variety of users are accumulated in the memory 32 of the server 30. It may be sufficient that the waveforms of the brain activities and other equivalent data are stored in the memory 32 to enable the specified users to extract the waveforms of the brain activities.

In S102, the user terminal 20 extracts signals of variation components of the bloodflow rate and signals of pulse wave components from the detection values (the waveforms of the brain activities) received from the head region attaching device 10. The user terminal 20 filters the received waveforms of the brain activities through a first bandpass filter, thereby extracting the variation components of the bloodflow rate from the waveforms of the brain activities. The bandpass filter is a filter for extracting signals of a predetermined frequency band. The frequency band of the signals passing through the first bandpass filter ranges from, e.g., 0.05 Hz to 0.14 Hz. A wave on the order of 0.1 Hz, which passes through the first bandpass filter, is called Mayer wave. The variation components of the bloodflow rate are defined as a quantity of the blood flowing through the blood vessels of the brain for unit time. The user terminal 20 filters the received waveforms of the brain activities through a second bandpass filter, thereby extracting the signals of the pulse wave components. A frequency band of the signals passing through the second bandpass filter is higher than the frequency band of the signals passing through the first bandpass filter. The frequency band of the signals passing through the second bandpass filter ranges from, e.g., 0.8 Hz to 2.0 Hz. The pulse wave components are components of waves formed by pulsations. The user terminal 20 stores, in the memory 22, the signals of the variation components of the bloodflow rate and the signals of the pulse wave components.

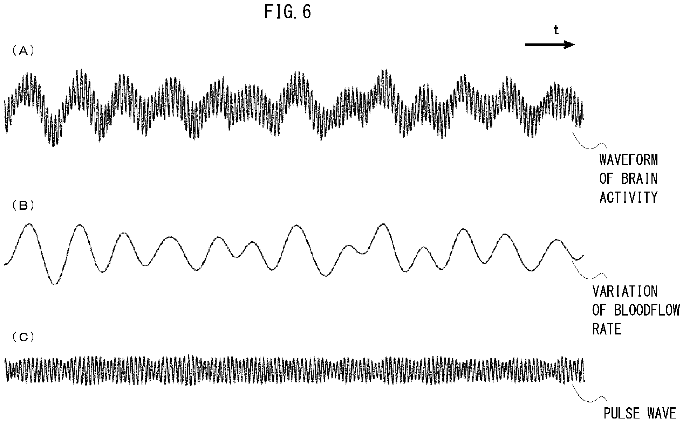

FIG. 6 is a graphic chart illustrating examples of the waveforms of the brain activities, the signals of the variation components of the bloodflow rate and the signals of the pulse wave components. In FIG. 6, a crosswise direction corresponds to a direction of a time base. FIG. 6 (A) is a graph depicting an example of the waveforms of the brain activities, which are given from the detection values. FIG. 6 (B) is a graph depicting the signals of the variation components of the bloodflow rate, which are extracted from the waveforms of the brain activities. The frequency band of the signals passing through the first bandpass filter is equal to or larger than 0.05 Hz but equal to or smaller than 0.14 Hz. FIG. 6(C) is a graph depicting the signals of the pulse wave components, which are extracted from the waveforms of the brain activities. The frequency band of the signals passing through the second bandpass filter is equal to or larger than 0.8 Hz but equal to or smaller than 2.0 Hz.

In S103, the user terminal 20 extracts an envelope of the signals of the pulse wave components extracted from the waveforms of the brain activities in S102. The envelope of the signals of the pulse wave components represents time variations (variation components of pulse amplitude) of amplitudes of the pulse wave components. The user terminal 20 lowpass-filters the signals of the pulse wave components by squaring these signals of the pulse wave components to extract the signals of the frequency lower than 0.8 Hz, and obtains the envelope (the signals of the variation components of the pulse amplitudes) by further taking a square root of the acquired signals . The lowpass filter is a filter for extracting the signals lower than a predetermined frequency. The user terminal 20 may also obtain the envelope (the signals of the variation components of the pulse amplitudes) of the signals of the pulse wave components from the signals of the pulse wave components by using Hilbert transform. The user terminal 20 may also obtain the signals of the variation components of the pulse amplitudes as the envelope of the signals of the pulse wave components by other methods . The user terminal 20 stores the obtained signals of the variation components of the pulse amplitudes in the memory 22.

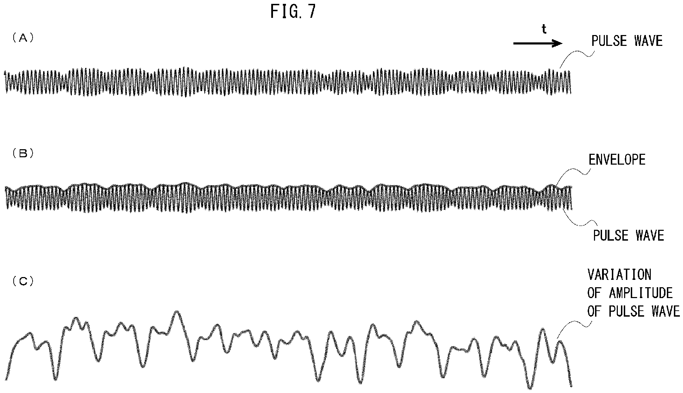

FIG. 7 is a graphic chart illustrating examples of the signals of the pulse wave components and the signals of the variation components of the pulse amplitudes. In FIG. 7, the crosswise direction is the direction of the time-base. FIG. 7 (A) is a graph depicting the signals of the pulse wave components . FIG. 7(B) is a graph depicting the signals of the pulse wave components and the envelope of the signals of the pulse wave components. FIG. 7(C) is a graph depicting the envelope (the signals of the variation components of the pulse amplitudes) of the signals of the pulse wave components . A vertical direction of the envelope in FIG. 7(B) is enlarged in the signals of the variation components of the pulse amplitudes in FIG. 7(C).

In S104, the user terminal 20 displays, on a display means of the output unit 27, the signals of the variation components of the bloodflow rate and the signals of the variation components of the pulse amplitudes that are stored in the memory 22 in a way that arranges these signals according to the time-base. The arrangement according to the time-base connotes displaying the signals by setting the detection time along the time-base. The user of the user terminal 20 is recognizable of the variations of the pulse amplitude (i.e., recognizable of how the sympathetic nerves are activated) when the bloodflow rate varies by watching the signals of the variation components of the bloodflow rate and the signals of the variation components of the pulse amplitudes, which are displayed on the display means.

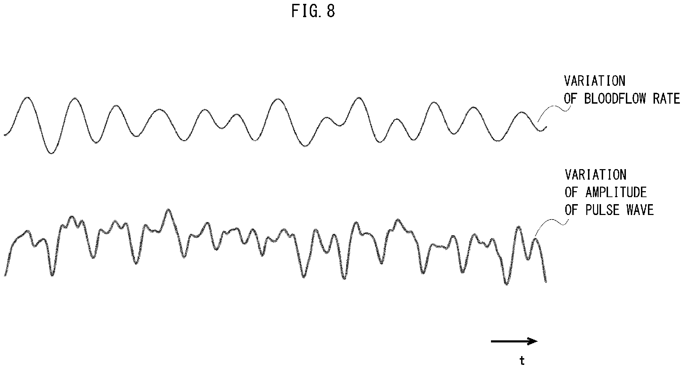

FIG. 8 is a graphic chart depicting examples of the signals of the variation components of the bloodflow rate and the signals of the variation components of the pulse amplitudes, which are outputted to the user terminal. In FIG. 8, the crosswise direction corresponds to the time-base. An upper graph of FIG. 8 is a graph depicting time variations of the bloodflow rate. A lower graph of FIG. 8 is a graph depicting time variations of the pulse amplitude. The user of the user terminal 20 is enabled to perform training instanced by the cogitation so that phases of the two graphs become coincident with desired phases while seeing the two graphs.

The user terminal 20 may plot the variation components of the bloodflow rate and the variation components of the pulse amplitudes per elapse time when measuring the waveforms of the brain activities, in which the variation components of the bloodflow rate are given along an axis of abscissa of orthogonal coordinates, while the variation components of the pulse amplitudes are given along an axis of ordinate thereof. A phase difference between the variation component of the bloodflow rate and the variation component of the pulse amplitude is "0", in which case a line connecting plotted points becomes a straight line connecting a left lower portion to a right upper portion. When the phase difference between the variation component of the bloodflow rate and the variation component of the pulse amplitude is $\pm\pi/2$, a line connecting the plotted points becomes circular. When the phase difference between the variation component of the bloodflow rate and the variation component of the pulse amplitude is $\pi$, a line connecting the plotted points becomes a straight line connecting a left upper portion to a right lower portion. The user of the user terminal 20 is enabled to perform training instanced by the cogitation so that this line takes a desired shape (the phase becomes the desired phase) while seeing the line connecting the plotted points.

The user terminal 20 may display, on the display means of the output unit 27, time variations of the difference (phase difference) between the phase (instantaneous phase) of the signals of the variation components of the bloodflow rate and the phase (instantaneous phase) of the signals of the variation components of the pulse amplitudes, which are stored in the memory 22. The user of the user terminal 20 is recognizable of the phase difference between the variations of the bloodflow rate and the variations of the pulse amplitude (i.e., recognizable of how the sympathetic nerves are activated) by watching the time variations of the phase difference, which are displayed on the display means. The user terminal 20 may display the time variations of the respective phases on the display unit 25 and/or the display means of the output unit 27.

Herein, the user terminal 20 calculates the phase (instantaneous phase) of the waveform of the variation component of the bloodflow rate or the variation component of the pulse amplitude by an expression given below.

[Mathematical Expression 1]

$$\phi(t) = \tan^{-1}\left[\frac{H[f(t)]}{f(t)}\right] \qquad (1)$$

where $\varphi(t)$ is the time variation of the phase (instantaneous phase), and f(t) represents the waveform of the variation component of the bloodflow rate or the variation component of the pulse amplitude. A symbol H[·] represents the Hilbert transform. The phase difference is the difference between the phase (instantaneous phase) of the signal of the variation component of the bloodflow rate and the phase of the signal of the variation component of the pulse amplitude.

The user terminal 20 acquires the waveforms of the brain activities of a predetermined user (another user) from the server 30, and may calculate the signal of the variation component of the bloodflow rate and the signal of the variation component of the pulse amplitude from the waveforms of the brain activities of this user. The predetermined user is a target person exemplified by an exemplary person, a teacher, a coach, an instructor, a successful person or a senior of the predetermined behaviors (cogitation and other equivalent behaviors), or a healthy person. The user terminal 20 arrange, e.g., in a comparable manner, the calculated signal of the variation component of the bloodflow rate of the predetermined user and the calculated signal of the variation component of the pulse amplitude, and further the signal of the variation component of the bloodflow rate and the signal of the variation component of the pulse amplitude that are based on the waveforms of the brain activities of the user of the user terminal 20, and thus displays these arranged signals on the display unit 25 and/or the display means of the output unit 27. The user of the user terminal 20 is able to perform the training of the cogitation and other behaviors) while confirming the signals of the predetermined user and the self-signals, which are displayed on the display means. The user of the user terminal 20 strives for approaching the signals of the target person with respect to the cogitation and other equivalent behaviors, thereby enabling improvements of a skill and other equivalent capabilities of the user of the user terminal 20.

(Operations and Effects of Embodiment)

The user terminal 20 acquires the waveforms of the brain activities when the user takes the predetermined behavior instanced by the cogitation. The user terminal 20 extracts the signals of the variations of the bloodflow rate and the signals of the variations of the pulse wave from the acquired waveforms of the brain activities. The user terminal 20 extracts the signals of the variations of the pulse amplitude from the signals of the variations of the pulse wave. The user terminal 20 displays, on the display means, the graphs (the waveforms themselves of the time variations of the signal, and the time variations and other equivalent variations of the phase difference), which are based on the signals of the variations of the bloodflow rate and the signals of the variations of the pulse amplitude. The user of the user terminal 20 is enabled to perform the training of the cogitation and other equivalent behaviors so as to attain the predetermined state while seeing the graphs displayed on the display means. The pulse amplitude varies corresponding to the state of how the sympathetic nerves are activated, and hence, according to the system of the embodiment, it is feasible to observe the state of how the bloodflow rate of the user varies and the state of how the sympathetic nerves are activated.

The user terminal 20 acquires the waveforms of the brain activities of another user, and is thereby enabled to display the graph based on the signals of the variation components of the bloodflow rate and the graph based on the signals of the variation components of the pulse amplitude. It is also feasible to display the graphs of the user of the user terminal 20 and the graphs of another user on a side-by-side basis to make these graphs comparable with each other. The user of the user terminal 20 is able to perform the training of the predetermined behavior (the cogitation and other equivalent behaviors) while confirming the signals of another user and the self-signals, which are displayed on the display means. The user of the user terminal 20 performs the training so as to approach the signals of another user becoming the target person with respect to the predetermined behavior (the cogitation and other equivalent behaviors), and is thereby enabled to make the skill and other equivalent capabilities of the user of the user terminal 20 approach to those of another user becoming the target person.

<Non-Transitory Computer Readable Recording Medium>

A program configured to cause a computer, other machines and apparatuses (which will hereinafter be referred to as the computer and other equivalent apparatuses) to attain any one of the functions, can be recorded on a non-transitory recording medium readable by the computer and other equivalent apparatuses. The computer and other equivalent apparatuses are made to read and execute the program on this non-transitory recording medium, whereby the function thereof can be provided.

Herein, the non-transitory recording medium readable by the computer and other equivalent apparatuses connotes a non-transitory recording medium capable of accumulating information instanced by data, programs and other equivalent information electrically, magnetically, optically, mechanically or by chemical action, which can be read from the computer and other equivalent apparatuses. Components instanced by the CPU and the memory configuring the computer are provided within such a non-transitory recording medium, in which the CPU may be made to run the program.

Among these non-transitory recording mediums, the mediums removable from the computer and other equivalent apparatuses are exemplified by a flexible disc, a magneto-optic disc, a CD-ROM, a CD-R/W, a DVD, a DAT, an 8 mm tape, and a memory card.

A hard disc, a Read-Only Memory (ROM) and other equivalent recording mediums are given as the non-transitory recording mediums fixed within the computer and other equivalent apparatuses.

[Description of the Reference Numerals and Symbols]

| | |
|---|---|
| 10 | head region attaching device |
| 11 | control unit |
| 13 | wireless communication unit |
| 115 | sensor |
| 125 | sensor |
| 20 | user terminal |
| 21 | CPU |
| 22 | memory |
| 23 | wireless communication unit |
| 24 | public network communication unit |
| 25 | display unit |
| 26 | operation unit |
| 27 | output unit |
| 28 | image capturing unit |

29      positioning unit
30      server
31      CPU
32      memory
34      public network communication unit
35      display unit
36      operation unit
37      output unit

**Claims**

1.  An information processing device comprising:

    a receiving unit to receive a detection value detected by a head region attaching device, attached to a head region of a user, to detect a bloodflow rate of the head region;
    a calculation unit to extract a first signal by filtering the detection value with a first filter to extract a signal of a first frequency band, to extract a second signal by filtering the detection value with a second filter to extract a signal of a second frequent band higher than the first frequency band, and to extract a third signal as an envelope of the second signal from the second signal; and
    an output unit to output the first signal and the third signal by being associated with each other.

2.  The information processing device according to claim 1, wherein the first signal is a signal corresponding to a variation of bloodflow rate of the head region of the user, and the second signal is a signal corresponding to a variation of pulse amplitude of the user.

3.  The information processing device according to claim 1 or 2, wherein the first filter is a bandpass filter to select a signal of a frequency band equal to or larger than 0.05 Hz but equal to or smaller than 0.14 Hz, and
    the second filter is a bandpass filter to select a signal of a frequency band equal to or larger than 0.8 Hz but equal to or smaller than 2.0 Hz.

4.  An information processing method by which a computer executes:

    extracting a first signal by filtering a detection value, detected by a head region attaching device, attached to a head region of a user, to detect a bloodflow rate of the head region, with a first filter to extract a signal of a first frequency band, extracting a second signal by filtering the detection value with a second filter to extract a signal of a second frequent band higher than the first frequency band, and extracting a third signal as an envelope of the second signal from the second signal; and
outputting the first signal and the third signal by being associated with each other.

5.  An information processing program to make a computer execute:

    extracting a first signal by filtering a detection value, detected by a head region attaching device, attached to a head region of a user, to detect a bloodflow rate of the head region, with a first filter to extract a signal of a first frequency band, extracting a second signal by filtering the detection value with a second filter to extract a signal of a second frequent band higher than the first frequency band, and extracting a third signal as an envelope of the second signal from the second signal; and
    outputting the first signal and the third signal by being associated with each other.

# FIG. 1

SERVER

~ 30

N2

USER
TERMINAL

~ 20

N1

HEAD REGION
ATTACHING DEVICE

~ 10

# FIG. 2

# FIG. 3

```
┌─────────────────────────────────────────┐
│  ┌──────────┐    ┌──────────────┐        │
│  │   CPU    │────│    MEMORY    │        │
│  └──────────┘    └──────────────┘        │
│         21              22                │
│     ┌─────────────────────────┐  23      │
│     │       WIRELESS          │          │
│     │  COMMUNICATION UNIT     │  24      │
│     └─────────────────────────┘          │
│     ┌─────────────────────────┐          │
│     │    PUBLIC NETWORK        │          │
│     │  COMMUNICATION  UNIT     │          │
│     └─────────────────────────┘          │
│     ┌─────────────────────────┐          │
│     │      DISPLAY UNIT        │  25      │
│     └─────────────────────────┘          │
│     ┌─────────────────────────┐          │
│     │     OPERATION UNIT       │  26      │
│     └─────────────────────────┘          │
│     ┌─────────────────────────┐          │
│     │      OUTPUT UNIT         │  27      │
│     └─────────────────────────┘          │
│     ┌─────────────────────────┐          │
│     │    IMAGE CAPTURING       │  28      │
│     │         UNIT             │          │
│     └─────────────────────────┘          │
│     ┌─────────────────────────┐          │
│     │   POSITIONNING UNIT      │  29      │
│     └─────────────────────────┘          │
│     ┌─────────────────────────┐          │
│     │   PHYSICAL SENSOR UNIT   │  2A      │  20
│     └─────────────────────────┘          │
│  USER TERMINAL                           │
└─────────────────────────────────────────┘
```

FIG. 4

```
┌─────────────────────────────────────────┐
│  ┌──────────┐      ┌──────────┐          │
│  │   CPU    │──────│  MEMORY  │          │
│  └──────────┘      └──────────┘          │
│        ╰─31              ╰─32            │
│    │                                     │
│    │   ┌──────────────────────┐          │
│    │   │   PUBLIC NETWORK      │          │
│    ├───│   COMMUNICATION       │          │
│    │   │       UNIT            │          │
│    │   └──────────────────────┘          │
│    │                        ╰─ 34        │
│    │   ┌──────────────────┐   35         │
│    ├───│   DISPLAY UNIT    │──╯           │
│    │   └──────────────────┘              │
│    │   ┌──────────────────┐              │
│    ├───│  OPERATION UNIT   │── 36        │
│    │   └──────────────────┘              │
│    │   ┌──────────────────┐              │
│    ├───│   OUTPUT UNIT     │── 37        │
│    │   └──────────────────┘              │
│    │                                     │
│  SERVER                                  │
└─────────────────────────────────────────┘
                        ╰─ 30
```

SERVER

30

# FIG. 5

```
         ┌─────────────────────────┐
         │          START          │
         └─────────────────────────┘
                      │
                      ▼
  ┌───────────────────────────────────────────┐
  │      MEASURE STATE OF BRAIN ACTIVITY       │───S101
  └───────────────────────────────────────────┘
                      │
                      ▼
  ┌───────────────────────────────────────────┐
  │  EXTRACT VARIATION COMPONENT OF BLOODFLOW  │
  │    RATE AND COMPONENT OF PULSE WAVE        │───S102
  └───────────────────────────────────────────┘
                      │
                      ▼
  ┌───────────────────────────────────────────┐
  │   EXTRACT VARIATION COMPONENT OF AMPLITUDE │
  │ OF PULSE AMPLITUDE FROM COMPONENT OF PULSE WAVE│───S103
  └───────────────────────────────────────────┘
                      │
                      ▼
  ┌───────────────────────────────────────────┐
  │     OUTPUT COMPONENT OF BLOODFLOW RATE      │
  │ AND VARIATION COMPONENT OF AMPLITUDE OF PULSE WAVE│───S104
  └───────────────────────────────────────────┘
                      │
                      ▼
         ┌─────────────────────────┐
         │           END           │
         └─────────────────────────┘
```

FIG. 6

(A)

WAVEFORM
OF BRAIN
ACTIVITY

(B)

VARIATION
OF BLOODFLOW
RATE

(C)

PULSE WAVE

FIG. 7

(A)

t

PULSE WAVE

(B)

ENVELOPE

PULSE WAVE

(C)

VARIATION
OF AMPLITUDE
OF PULSE WAVE

FIG. 8

VARIATION
OF BLOODFLOW RATE

VARIATION
OF AMPLITUDE
OF PULSE WAVE

t

## INTERNATIONAL SEARCH REPORT

| International application No. |
|---|
| PCT/JP2016/078050 |

| A. CLASSIFICATION OF SUBJECT MATTER |
|---|
| *A61B5/026*(2006.01)i |

According to International Patent Classification (IPC) or to both national classification and IPC

| B. FIELDS SEARCHED |
|---|

Minimum documentation searched (classification system followed by classification symbols)
A61B5/026

Documentation searched other than minimum documentation to the extent that such documents are included in the fields searched

| Jitsuyo Shinan Koho | 1922–1996 | Jitsuyo Shinan Toroku Koho | 1996–2016 |
|---|---|---|---|
| Kokai Jitsuyo Shinan Koho | 1971–2016 | Toroku Jitsuyo Shinan Koho | 1994–2016 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| A | JP 2015-073835 A (Pioneer Corp.), 20 April 2015 (20.04.2015), paragraphs [0014] to [0078] (Family: none) | 1-5 |
| A | WO 2014/050299 A1 (Omron Healthcare Co., Ltd.), 03 April 2014 (03.04.2014), paragraphs [0020] to [0114] & US 2015/0196206 A1 paragraphs [0038] to [0136] & JP 2014-068826 A & CN 104684467 A | 1-5 |
| A | JP 2006-346164 A (Seiko Instruments Inc.), 28 December 2006 (28.12.2006), paragraphs [0009] to [0043] (Family: none) | 1-5 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 November 2016 (28.11.16) | 06 December 2016 (06.12.16) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000037558 A